# EUROPÄISCHE PATENTSCHRIFT

(11) **EP 2 314 240 B1**
(45) Veröffentlichungstag und Bekanntmachung des Hinweises auf die Patenterteilung: **14.08.2013**
(21) Anmeldenummer: 10186144.1
(22) Anmeldetag: 25.08.2003
(51) Int. Cl.: A61B 17/86

(54) **Knochenschraube mit Halteelement**
Bone screw with holding element
Vis à os avec élément de maintien

(30) Priorität: 04.10.2002 DE 10246386
(43) Veröffentlichungstag der Anmeldung: 27.04.2011
(62) Teilanmeldung aus: 03019180.3
(73) Patentinhaber: Biedermann Technologies GmbH & Co. KG, 78166 Donaueschingen (DE)
(72) Erfinder: Biedermann, Lutz, 78048, VS-Villingen (DE); Harms, Jürgen, 76227, Karlsruhe (DE)
(74) Vertreter: Prüfer & Partner GbR European Patent Attorneys

(56) Entgegenhaltungen:
- WO-A-02/38054
- DE-A1- 3 027 138
- FR-A1- 2 820 630
- US-A- 4 653 489
- US-A- 5 645 546
- US-A- 5 964 761
- US-A- 6 048 343
- US-A1- 2002 055 783
- W. KAISER, J.BURMESTER, V. LARCHER: "Zur Verfahrenswahl bei pertrochanTären Femurfrakturen", UNFALLCHIRURGIE, Bd. 25, Nr. 2, 1999, Seiten 50-54, XP002612374,

## Beschreibung

Die vorliegende Erfindung bezieht sich auf eine Knochenschraube, insbesondere auf eine Knochenschraube mit einem Gewindeabschnitt mit einem netzartigen Aufbau.

Eine derartige Knochenschraube ist aus der DE 100 55 891 Al bekannt. Sie beinhaltet einen Gewindeabschnitt, eine Spitze an dem einen Ende und einen Kopf zum Eingreifen mit einem Schraubendreher an dem anderen Ende. Der Gewindeabschnitt ist rohrförmig ausgebildet und seine Wandung weist eine Mehrzahl von Ausnehmungen auf.

Die bekannte Schraube kann z.B. bei der Behandlung von osteoporotischen Frakturen eingesetzt werden. Aufgrund der netzartigen Ausbildung der Schraube kann eine Fusion mit umgebendem Knochenmaterial stattfinden. Die Position der Fraktur im Knochen, die z.B. beim Hüftknochen oftmals weit von der Oberfläche entfernt ist, von der aus die Schraube eingeschraubt wird, erfordert zur sicheren Fixation eine präzise Positionierung der Knochenschraube und zum Teil auch eine mechanische Verbindung zu externen und/oder internen Verstrebungsimplantaten verbunden werden kann.

US 6,048,343 A offenbart eine Knochenschraube mit einem Kopf und einem Schaft und einem Adapter, der lösbar mit dem Kopf der Knochenschraube verbunden ist, um so durch den Adapter z.B. Knochenzement einzufüllen.

US 5, 964 761 A offenbart eine expandierbare Pedikelschraube mit einem Schaft und einer rohrförmigen Erweiterung, wobei die rohrförmige Erweiterung mit einer anderen Pedikelschraube verbunden werden kann

Der vorliegenden Erfindung liegt die Aufgabe zugrunde, eine Knochenschraube der oben beschriebenen Art und eine Knochenfixationseinrichtung mit verbesserten Eigenschaften und Einsatzmöglichkeiten bereitzustellen, die insbesondere präzise positionierbar ist und wahlweise auch mit externen und/oder internen Verstrebungsimplantaten.

Die Aufgabe wird gelöst durch eine Knochenschraube gemäß Anspruch 1 und eine Knochenfixationseinrichtung gemäß Anspruch 12. Weiterbildungen der Erfindung sind in den Unteransprüchen gekennzeichnet.

Die erfindungsgemäße Knochenschraube weist insbesondere den Vorteil auf, dass sie im Knochen an die erforderliche Stelle versenkbar ist. Somit erfüllt sie ihre Funktion als Zugelement, ohne dass ein Teil aus dem Knochen heraussteht.

Ferner lassen sich Knochenzement oder Wirkstoffe mittels der Knochenschraube präzise platzieren.

Weitere Merkmale und Zweckmäßigkeiten der Erfindung ergeben sich aus der Beschreibung von Ausführungsbeispielen anhand der beigefügten Zeichnungen. Von den Figuren zeigen:
- Fig. 1: eine Explosionsdarstellung des modularen Aufbaus einer Knochenschraube gemäß einer ersten bis drit- ten Ausführungsform;
- Fig. 2: eine Darstellung der in Fig. 1 gezeigten Knochen- schraube in zusammengesetztem Zustand;
- Fig. 3: eine Schrittfolge zum Befestigen einer äußeren Platte an einem Knochen.
- Fig. 4: eine Darstellung einer Kombination der Knochen- schraube mit einem Marknagel.
- Fig. 5: eine Darstellung einer Knochenschraube gemäß einer vierten Ausführungsform;

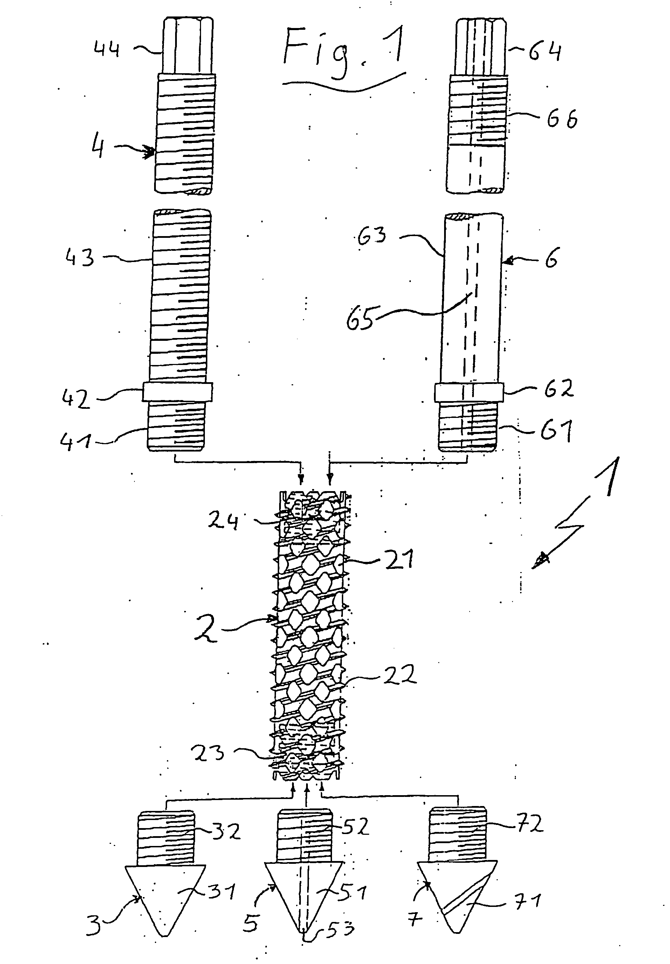

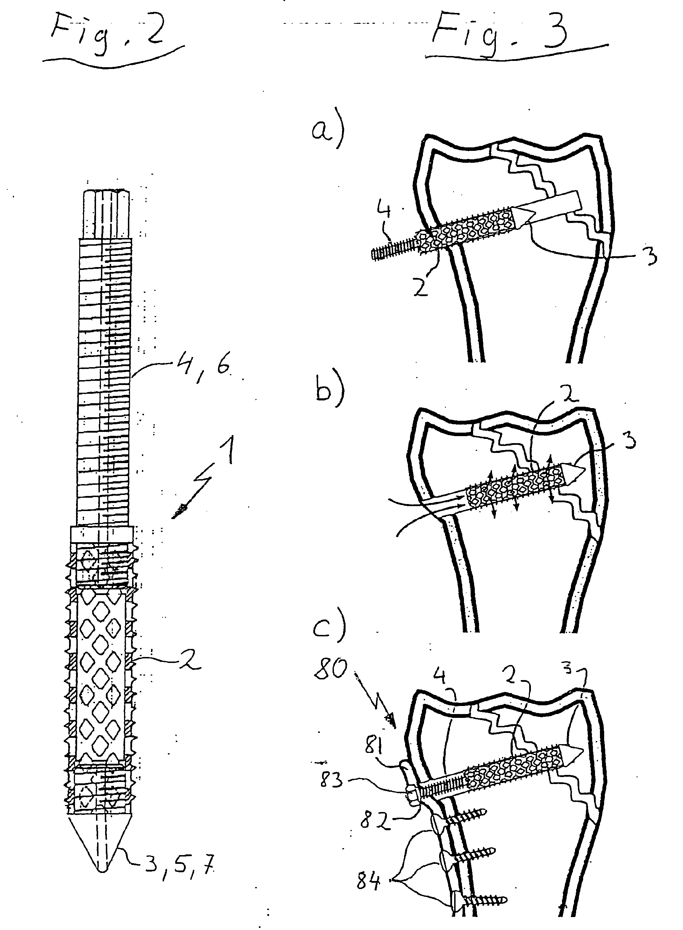

Im folgenden wird mit Bezug auf Fig. 1 und 2 eine Knochenschraube nach einer Ausführungsform beschrieben. Dabei ist in Fig. 2 zur Verbesserung der Übersichtlichkeit ein Teil der Knochenschraube im Schnitt dargestellt.

Eine erste Ausführungsform der in Fig. 1 und 2 dargestellten modularen Knochenschraube 1 beinhaltet einen Gewindeabschnitt 2, eine Spitze 3 und ein Halteelement 4.

Der Gewindeabschnitt 2 ist rohrförmig ausgebildet und weist in seiner Wandung eine Vielzahl von Ausnehmungen 21 auf, die in dem gezeigten Ausführungsbeispiel rautenförmig ausgebildet sind. Die Rauten sind derart ausgerichtet, dass sich ihre Symmetrieachse jeweils parallel zu der Symmetrieachse des Rohres erstreckt. Die Rauten sind ferner gegeneinander versetzt angeordnet.

Auf der Außenwandung ist ein sogenanntes Knochengewinde 22 vorgesehen, welches in seiner Form den üblichen Knochen-schrauben entspricht.

Auf der Innenwandung weist der rohrförmige Gewindeabschnitt 2 an seinen beiden Enden je ein metrisches Innengewinde 23, 24 auf.

Die Spitze 3 beinhaltet den eigentlichen Spitzenteil 31 und einen Schaft 32. In dem gezeigten Ausführungsbeispiel weist der Schaft 32 ein Außengewinde auf, das dem Innengewinde 23 des rohrförmigen Gewindeabschnitts 2 entspricht, zum Verbinden der Spitze 3 mit dem Gewindeabschnitt 2 wie in Fig. 2 dargestellt.

Das Halteelement 4 weist an einem ersten Ende einen ersten Abschnitt 41 mit einem Außengewinde auf, das dem Innengewinde 23 des rohrförmigen Gewindeabschnitts 2 entspricht, zum Verbin-den des Halteelements 4 mit dem Gewindeabschnitt 2 wie in Fig. 2 dargestellt.

An dem Ende des Gewindeabschnitts 41 ist ein Anschlag 42 in Form einer Schulter bzw. eines ringförmigen Vorsprungs vorgesehen. Durch diesen Anschlag wird der Einschraubweg beim Einschrauben begrenzt. Wie insbesondere aus Fig. 2 ersichtlich, entspricht der Außendurchmesser des ringförmigen Vorsprungs 42 dem Außendurchmesser des rohrförmigen Gewindeabschnitts 2, so dass er beim Einschrauben der Knochenschraube in der durch diese geschaffenen Öffnung kein Hindernis darstellt.

Das Halteelement weist weiterhin einen stabförmigen Abschnitt 43 auf, der wie in Fig. 2 dargestellt in eingeschraubtem Zustand über das zweite Ende des Gewindeabschnitts 2 hinausragt. Der stabförmige Abschnitt 43 ist vorzugsweise durchgehend mit einem metrischen Außengewinde versehen.

Die axiale Länge des stabförmigen zweiten Abschnitts 43 ist bevorzugt größer als die des ersten, in den Gewindeabschnitt 2 einzuschraubenden Abschnitts 41. Sie ist in Abhängigkeit von dem Einsatzort der Knochenschraube so gewählt, dass der rohrförmige Gewindeabschnitt 2 an die erforderliche Stelle in dem Knochen versenkbar ist.

An dem seinem dem Abschnitt 41 gegenüberliegenden freien Ende weist das Halteelement ein Angriffselement für ein Einschraubwerkzeug in Form eines Außensechskantabschnitts 44 auf.

Der Gewindeabschnitt 2, die Spitze 3 und das Halteelement 4 sind vorzugsweise aus Titan geformt. Es kann jedoch auch ein anderes biokompatibles Material verwendet werden.

Im folgenden wird mit Bezug auf Fig. 2 und 3 der Einsatz der Knochenschraube nach der ersten Ausführungsform beschrieben. Die Knochenschraube 1 wird dabei z.B. in das Ende eines Röhrenknochens eingesetzt, der durch Osteoporose von innen her geschwächt ist, z.B. in den Schenkelhals eines Oberschenkelknochens.

Zunächst wird die Spitze 3 in den rohrförmigen Gewindeabschnitt 2 eingeschraubt. Als nächstes wird das Halteelement 4 in den Gewindeabschnitt 2 eingeschraubt. Dadurch erhält man den in Fig. 2 dargestellten Aufbau.

Wie in Fig. 3 a) dargestellt, wird als nächstes die aus Gewindeabschnitt 2, Spitze 3 und Halteelement 4 zusammen-gesetzte Knochenschraube 1 in den Knochen hinein geschraubt. Dabei kann der Gewindeabschnitt 2 tief in den Knochen versenkt und dabei präzise an der Frakturstelle positioniert werden. Anschließend wird das Halteelement 4 wieder aus dem Gewindeabschnitt 2 herausgeschraubt.

Wie in Fig. 3 b) dargestellt, wird in einem weiteren Schritt in das Innere des Gewindeabschnitts 2 ein Füllmaterial wie z.B. Knochenzement oder Ähnliches und/oder ein medikamentöser oder wachstumsfördernder Wirkstoff eingespritzt. Im Gegensatz zu dem direkten Einspritzen sorgt der Gewindeabschnitt 2 für eine genauere Positionierung des Einspritzkanals und für eine gleichmäßigere Verteilung des eingespritzten Materials, das durch die Ausnehmungen 21 in die benachbarten Abschnitte des Knochens austreten kann. Dadurch wird die Fixierung des Knochens durch die Schraube weiter verbessert.

Die versenkte Knochenschraube dient als Verstärkung für die z.B. durch Osteoporose geschwächten Knochenbälkchen. Durch die Ausnehmungen 21 kann der Knochen in die Schraube einwachsen. Eine Stabilisierung der Frakturstelle des geschwächten Knochens erfolgt somit als Kombination von Zugentlastung und Fusion.

Fig. 3 c) zeigt eine Fixationseinrichtung 80 zum Fixieren des Knochens mit Hilfe einer äußeren Fixationsplatte. Dabei wird das Halteelement 4 nach dem Einspritzen des Füllmaterials wieder in den Gewindeabschnitt 2 eingeschraubt. Eine Platte 81 mit einer Ausnehmung 82 wird so an den Knochen angelegt, dass das freie Ende des Halteelements 4 durch die Ausnehmung 82 ragt, und durch Aufschrauben einer Mutter 83 auf das Außenge-winde des stabförmigen Abschnitts 43 des Halteelements 4 fest mit der Knochenschraube 1 verbunden. Weitere Knochenschrauben 84 dienen zum stabilen Fixieren der Platte 81 an dem Knochen. Der stabförmige Abschnitt 43 kann hierzu auf die erforderliche Länge gekürzt werden, die so bemessen ist, dass er nicht wesentlich über die Mutter 83 hervorsteht.

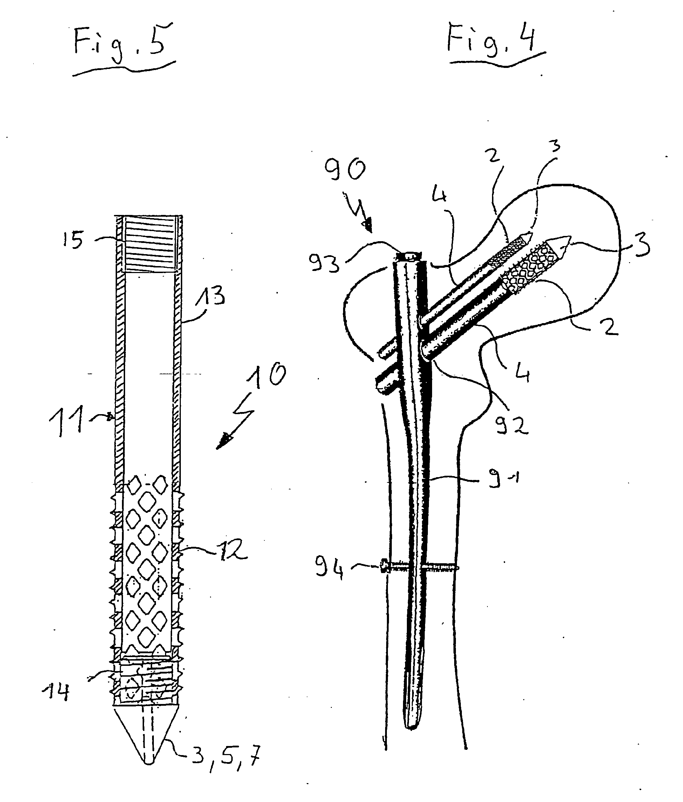

Fig. 4 zeigt ein weiteres Anwendungsbeispiel für die Knochenschraube nach der ersten Ausführungsform. Bei der Fixationseinrichtung 90 erfolgt die Fixierung des Knochens nicht Hilfe einer äußeren Fixationsplatte, sondern mit Hilfe eines in den Knochen eingebrachten Marknagels 91. Der Marknagel 91 weist zumindest eine Ausnehmung 92 auf, durch die zumindest ein Halteelement 4 einer Knochenschraube 1 hindurchragt. In dem dargestellten Ausführungsbeispiel sind es zwei Knochenschrauben 1 mit unterschiedlichen Durchmessern. Eine Verschlussschraube 93 verschließt den Marknagel nach außen, und eine Verriege-lungsschraube (94) verhindert eine Bewegung in Längsrichtung.

Eine zweite Ausführungsform der in Fig. 1 dargestellten modularen Knochenschraube 1 unterscheidet sich von der ersten Ausführungsform darin, dass anstelle der Spitze 3 eine kanulierte Spitze 5 und anstelle des Halteelements 4 ein kanuliertes Halteelement 6 vorgesehen ist. Nur die Ausführungsformen mit kanuliertem Halteelement 6 fallen in den Schutzbereich der Ansprüche.

Die kanulierte Spitze 5 unterscheidet sich von der Spitze 3 dadurch, dass zusätzlich eine koaxiale Bohrung 53 zum Hindurchleiten eines Wirkstoffs vorgesehen ist.

Das kanulierte Halteelement 6 unterscheidet sich von dem Halteelement 4 dadurch, dass zusätzlich eine koaxiale Bohrung 65 in vorgesehen ist und dass der stabförmige Abschnitt 63 nicht wie der stabförmige Abschnitt 43 bei der ersten Ausführungsform über seine gesamte Länge mit einem metrischen Gewinde versehen ist, sondern nur in einem Teilabschnitt 66.

Alle anderen Merkmale stimmen mit der ersten Ausführungsform überein, und auch der Einsatz erfolgt in der gleichen Weise.

Diese Ausführungsform hat den Vorteil, dass sich auch ohne Herausschrauben des Halteelements und auch im Nachhinein z.B. mittels einer Spritze ein medikamentöser Wirkstoff präzise an die gewünschte Stelle einbringen lässt.

Eine dritte Ausführungsform der in Fig. 1 dargestellten modularen Knochenschraube 1 unterscheidet sich von der ersten und zweiten Ausführungsform darin, dass anstelle der Spitze 3 eine selbstschneidende Spitze 7 vorgesehen ist. Alle anderen Merkmale stimmen mit der ersten bzw. zweiten Ausführungsform überein, und auch der Einsatz erfolgt in der gleichen Weise.

Durch die selbstschneidende Spitze wird das Einbringen der Knochenschraube erleichtert.

Eine in Fig. 5 dargestellte vierte Ausführungsform unterscheidet sich von der in Fig. 1 und 2 dargestellten ersten bis dritten Ausführungsform in der Form des Gewindeabschnitts.

Die Knochenschraube 10 nach der vierten Ausführungsform beinhaltet einen Schraubenabschnitt 11 und eine Spitze. Der Schraubenabschnitt 11 ist rohrförmig ausgebildet und besteht aus einem Gewindeabschnitt 12 und einem Abschnitt 13. Als Spitze kann jede der in der ersten bis dritten Ausführungsform beschriebenen Spitzen 3, 5 und 7 verwendet werden.

Der Gewindeabschnitt 12 weist wie der bei der ersten Ausführungsform beschriebene Gewindeabschnitt 2 in seiner Wandung eine Vielzahl von Ausnehmungen 21 und auf seiner Außenwandung ein Knochengewinde 22 auf.

Der Abschnitt 13 ist dagegen knochengewindefrei ausgebildet. In dem dargestellten Ausführungsbeispiel sind in dem knochengewindefreien Abschnitt 13 in der Wandung keine Ausnehmungen ausgebildet. Es können jedoch auch hier Ausnehmungen vorgesehen sein.

Auf der Innenwandung weist der rohrförmige Schraubenabschnitt 11 an seinen beiden Enden je ein metrisches Innengewinde 14, 15 auf.

Die axiale Länge des knochengewindefreien Abschnitts 13 ist in Abhängigkeit von dem Einsatzort der Knochenschraube so gewählt, dass der Gewindeabschnitt 12 an die erforderliche Stelle in dem Knochen versenkbar ist. Sie kann insbesondere auch größer sein als die axiale Länge des Gewindeabschnitts 12.

Der rohrförmige Schraubenabschnitt 11 ist vorzugsweise aus Titan geformt. Es kann jedoch auch ein anderes biokompatibles Material verwendet werden.

Auch der Einsatz der vierten Ausführungsform erfolgt in der gleichen Weise wie bei der ersten bis dritten Ausführungsform. Zum Eindrehen der Knochenschraube 10 kann auf das Innengewinde 15 ein Halteelement 4, 6 aufgeschraubt werden, das später wieder entfernt wird oder verbleibt, oder ein Kopf mit einem Angriffselement für ein Einschraubwerkzeug. Das freie Ende des Abschnitts 13 ohne Knochengewinde kann auch selber ein Angriffselement für ein Einschraubwerkzeug aufweisen wie z.B. einen Kreuzschlitz.

Einige der beschriebenen Ausführungsformen stellen lediglich Beispiele für die Ausführung der Erfindung dar. Gewindeabschnitt, Spitze und Halteelement können nicht nur wie in den Ausführungsformen beschrieben miteinander kombiniert werden, sondern in beliebiger Weise. Dieser modulare Aufbau ermöglich eine Anpassung an die unterschiedlichsten Anforderungen.

Der Anschlag zum Begrenzen des Einschraubwegs des Halteelements 4, 6 in den Gewindeabschnitt 2 kann statt an dem Halteelement 4, 6 auch in dem rohrförmigen Gewindeabschnitt 2 vorgesehen sein, z.B. am Ende des Innengewindes 24.

Das Halteelement 4, 6 kann so ausgebildet sein, dass der stabförmige Abschnitt 43, 63 wie bei der ersten Ausführungsform über seine ganze Länge ein Außengewinde aufweist oder wie bei der zweiten Ausführungsform nur in einem Teilabschnitt. Der stabförmige Abschnitt 43, 63 kann aber auch gänzlich ohne Außengewinde ausgebildet sein. Der Durchmesser des stabförmigen Abschnitts 43, 63 kann wie in Fig. 1 und 2 dargestellt derselbe sein wie der des ersten Abschnitts 41, 61 des Halteelements 4, 6, er kann aber auch einen anderen, vorzugsweise kleineren Durchmesser aufweisen.

Auch der Halteabschnitt 14 nach der vierten Ausführungsform kann über seine ganze Länge ein Außengewinde aufweisen oder wie bei der zweiten Ausführungsform nur in einem Teilabschnitt.

Anstelle des Außensechskantabschnitts 44, 64 kann auch ein beliebiges anderes Angriffselement für ein Einschraubwerkzeug vorgesehen sein. So kann das freie Ende des Halteelement 4, 6 z.B. auch eine Ausnehmung für einen Inbusschlüssel aufweisen. Das Halteelement 4, 6 kann auch ohne Angriffselement 44, 64 für ein Einschraubwerkzeug ausgebildet sein. Das Einschrauben der Knochenschraube erfolgt dann mit einem geeigneten Werkzeug, das an dem stabförmigen Abschnitt 43 angreift.

Die Gewindeabschnitte 23, 24, 41, 43, 61, 66, 14 und 15 sind in den Ausführungsformen als metrisch beschrieben. Alternativ dazu können die Gewinde auch zöllig oder in einer anderen geeigneten Form ausgebildet sein.

Anstelle der Innengewinde 23 und 24 bzw. 14 und 15 kann auch ein einziges Innengewinde ausgebildet sein, das sich über die gesamte Länge des Gewindeabschnitts 2 bzw. des Schraubenabschnitts 11 erstreckt. Das hat den Vorteil, dass das rohrförmige Gewindeabschnitt 2 auf jede beliebige Länge abgelängt werden kann, so dass Schrauben gewünschter Länge herstellbar sind und dadurch die Lagerhaltung wesentlich verringert werden kann.

Alternativ zu den rautenförmigen Ausnehmungen 21 können auch andere Öffnungsformen vorgesehen sein, insbesondere runde Öffnungen.

Die Spitze 3, 5, 7 kann nicht nur durch Einschrauben mit dem Gewindeabschnitt 2 bzw. 12 verbunden werden, sondern das erste Ende des Gewindeabschnitts 2 bzw. 12 und der Schaft 32, 52, 72 der Spitze 3, 5, 7 können ohne die jeweiligen Gewinde ausgebildet und in ihren Abmessungen so bestimmt sein, dass die Spitze 3, 5, 7 im Passsitz mit dem Gewindeabschnitt 2 bzw. 12 fest verbunden ist. Alternativ dazu kann die Spitze 3, 5, 7 auf eine beliebige andere Weise mit dem Gewindeabschnitt 2 bzw. 12 fest verbunden sein. Spitze und Gewindeabschnitt können auch einstückig ausgeführt sein.

Auch das Halteelement 4, 6 kann im Passsitz oder auf eine beliebige andere Weise mit dem Gewindeabschnitt 2 fest verbunden sein.

Alternativ zu der in Fig. 3 a) bis c) gezeigten Vorgehensweise kann der rohrförmige Gewindeabschnitt 2 vor dem Aufschrauben des Halteelements z.B. mit Knochenmaterial gefüllt werden. Anschließend wird die Schraube dann versenkt.

Die Schraube kann auch in dem versenkten Zustand verbleiben, ohne dass sie über das Halteelement mit einer Platte verbunden wird.

Die mit Bezug auf Fig. 3 beschriebene Kombination von Zugentlastung und Fusion kann natürlich nicht nur bei dem als Beispiel erwähnten Oberschenkelknochen durchgeführt werden, sondern bei beliebigen anderen Knochen, z.B. Tibia.

Die Knochenschraube 1, 10 ist nicht nur, wie in den Ausführungsbeispielen dargestellt, für das Einbringen in Röhrenknochen geeignet. Sie kann z.B. auch in Wirbeln oder anderen Knochen eingesetzt werden.

Das Halteelement 4, 6 kann nicht nur in Kombination mit dem rohrförmigen Gewindeabschnitt 2 verwendet werden, sondern auch mit einer beliebigen anderen Form von Knochenschrauben, die z.B. massiv oder kanuliert ausgebildet sein kann. Die Verbindung mit der Knochenschraube erfolgt in gleicher Weise wie die oben beschriebene Verbindung mit dem Gewindeabschnitt 2.

## Patentansprüche

1. Knochenschraube mit einem rohrförmig ausgebildeten Gewindeabschnitt (2) mit einer Spitze (3) an seinem ersten Ende und einem diesem gegenüberliegenden zweiten Ende,
wobei der rohrförmige Gewindeabschnitt (2) auf seiner Außenwandung ein Knochengewinde (22) aufweist und die Wandung des Gewindeabschnitts (2) eine Mehrzahl von Ausnehmungen (21) aufweist;
wobei die Knochenschraube
ein Halteelement (6) aufweist mit
einem ersten Abschnitt (61) zum Verbinden mit dem zweiten Ende des Gewindeabschnitts (2) und
einem stabförmigen zweiten Abschnitt (63), der in dem eingesetzten Zustand des Halteelements (6) über das zweite Ende des Gewindeabschnitts (2) hinausragt,
wobei ein Anschlag vorgesehen ist zum Begrenzen des Einsetzwegs des Halteelements (6) in den Gewindeabschnitt (2), wobei das Halteelement (6) kanuliert ausgebildet ist, **dadurch gekennzeichnet, dass** das Halteelement implantierbar ist.

2. Knochenschraube nach Anspruch 1 **dadurch gekennzeichnet, dass**
der rohrförmig ausgebildete Gewindeabschnitt (2) an seinem zweiten Ende einen Innengewindeabschnitt (24) aufweist und
der erste Abschnitt (61) des Halteelements (6) ein Außengewinde aufweist zum Einschrauben in den Innengewindeabschnitt (24).

3. Knochenschraube nach Anspruch 1, **dadurch gekennzeichnet, dass** die Verbindung zwischen dem Halteelement (6) und dem Gewindeabschnitt (2) in Form eines Presssitzes erfolgt.

4. Knochenschraube nach einem der Ansprüche 1 bis 3, **dadurch gekennzeichnet, dass** das Halteelement (6) an seinem freien Ende ein Element (64) aufweist zum Ein- oder Angreifen mit einem Einschraubwerkzeug.

5. Knochenschraube nach einem der Ansprüche 1 bis 4, **dadurch gekennzeichnet, dass** der Anschlag in Form einer Schulter (62) zwischen dem ersten Abschnitt (61) und dem zweiten Abschnitt (63) des Halteelements vorgesehen ist.

6. Knochenschraube nach einem der Ansprüche 1 bis 4, **dadurch gekennzeichnet, dass** der Anschlag in dem rohrförmigen Gewindeabschnitt (2) der Knochenschraube (1) vorgesehen ist.

7. Knochenschraube nach einem der Ansprüche 1 bis 6, **dadurch gekennzeichnet, dass** der stabförmige Abschnitt (63) des Halteelements (6) ein Außengewinde aufweist zum Aufschrauben einer Mutter (83) von dem freien Ende des Halteelements (6) her.

8. Knochenschraube nach Anspruch 2 und einem der Ansprüche 1 bis 7, **dadurch gekennzeichnet, dass**
der rohrförmig ausgebildete Gewindeabschnitt (2) auch an seinem ersten Ende einen Innengewindeabschnitt (23) aufweist oder der Innengewindeabschnitt sich über die gesamte Länge des rohrförmig ausgebildeten Gewindeabschnitts (2) erstreckt und
die Spitze (3, 5, 7) an dem ersten Ende des Gewindeabschnitts (2) in diesen Innengewindeabschnitt (23) einschraubbar ist.

9. Knochenschraube nach einem der Ansprüche 2 oder 8, **dadurch gekennzeichnet, dass** der Innengewindeabschnitt (23, 24) als metrisches oder zölliges Gewinde ausgebildet ist.

10. Knochenschraube nach einem der Ansprüche 1 bis 9, **dadurch gekennzeichnet, dass** die Spitze (7) als selbstschneidende Spitze ausgebildet ist.

11. Knochenschraube nach einem der Ansprüche 1 bis 10, **dadurch gekennzeichnet, dass** die Spitze (5) kanuliert ausgebildet ist.

12. Knochenfixationseinrichtung (80) mit zumindest einer Knochenschraube nach Anspruch 7 und einem der Ansprüche 1 bis 11,
einer Platte (81) zum Fixieren eines Knochens mit einer Ausnehmung (82), durch die das freie Ende des Halteelements (4, 6) hindurchführbar ist, und
einer Mutter (83) zum Aufschrauben auf das Halteelement (4, 6) von seinem freien Ende her und zum Fixieren der Platte (81) .

13. Knochenfixationseinrichtung (90) mit zumindest einer Knochenschraube nach einem der Ansprüche 1 bis 11, und
einem Marknagel (91) zum Fixieren eines Knochens mit einer Ausnehmung (92), durch die das freie Ende des Halteelements (4, 6) hindurchführbar ist.

## Claims

1. Bone screw with a thread section of tubular construction (2) with a tip (3) at its first end and an opposite second end,
wherein the tubular thread section (2) comprises a bone thread (22) on its outer wall and the wall of the thread section (2) comprises a plurality of recesses (21) ;
wherein the bone screw comprises a
holding element (6) with a first section (61) for connecting with the second end of the thread section (2) and
a rod-shaped second section (63) which, in the inserted state of the holding element (6), protrudes beyond the second end of the thread section (2),
wherein a stop is provided for limiting the distance of insertion of the holding element (6) into the thread section (2), wherein the holding element (6) is cannulated, **characterized in that** the holding element is implantable.

2. Bone screw according to claim 1, **characterized in that**
the thread section of tubular construction (2) comprises an internal thread section (24) at its second end and
and the first section (61) of the holding element (6) comprises an external thread for screwing into the internal thread section (24).

3. Bone screw according to claim 1, **characterized in that** the connection between the holding element (6) and the thread section (2) is made in the form of a press-fit.

4. Bone screw according to one of the claims 1 to 3, **characterized in that** the holding element (6) comprises an element (64) at its free end for engaging in or on with a screw-in tool.

5. Bone screw according to one of the claims 1 to 4, **characterized in that** the stop is provided in form of a shoulder (62) between the first section (61) and the second section (63) of the holding element.

6. Bone screw according to one of the claims 1 to 4, **characterized in that** the stop is provided in the tubular thread section (2) of the bone screw (1).

7. Bone screw according to one of the claims 1 to 6, **characterized in that** the rod-shaped section (63) of the holding element (6) comprises an external thread for screwing on a nut (83) from the free end of the holding element (6).

8. Bone screw according to claim 2 and one of the claims 1 to 7, **characterized in that**
the thread section of tubular construction (2) comprises an internal thread section (23) on its first end as well or the internal thread section extends over the whole length of the thread section of tubular construction (2) and the tip (3, 5, 7) at the first end of the thread section (2) is screwable into this internal thread section (23).

9. Bone screw according to one of the claims 2 or 8, **characterized in that** the internal thread section (23, 24) is designed as metric or inch thread.

10. Bone screw according to one of the claims 1 to 9, **characterized in that** the tip (7) is designed as a self-tapping tip.

11. Bone screw according to one of the claims 1 to 10, **characterized in that** the tip (5) is of cannulated construction.

12. Bone fixation device (80) with at least one bone screw according to claim 7 and one of the claims 1 to 11,
a plate (81) for fixing a bone with a recess (82), through which the free end of the holding element (4, 6) is guidable, and
a nut (83) for screwing onto the holding element (4, 6) from its free end and for fixing the plate (81).

13. Bone fixation device (90) with at least one bone screw according to one of the claims 1 to 11, and
a marrow nail (91) for fixing a bone with a recess (92), through which the free end of the holding element (4, 6) is guidable.

## Revendications

1. Vis à os, constituée d'un tronçon fileté (2) tubulaire avec une pointe (3) à sa première extrémité et une deuxième extrémité opposée à celle-ci,
le tronçon fileté (2) tubulaire présentant, sur sa paroi extérieure, un filetage à os (22) et la paroi du tronçon fileté (2) présentant une pluralité d'évidements (21) ;
ladite vis à os présentant un élément de maintien (6) avec un premier tronçon (61) à raccorder à la deuxième extrémité du tronçon fileté (2) et
un deuxième tronçon (63) en forme de tige, qui ressort de l'extrémité du tronçon fileté (2) en état de montage de l'élément de maintien (6),
une butée étant prévue pour limiter la course d'insertion de l'élément de maintien (6) dans le tronçon fileté (2),
l'élément de maintien (6) étant canulé,
**caractérisée en ce que** l'élément de maintien est implantable.

2. Vis à os selon la revendication 1, **caractérisée en ce que**
le tronçon fileté (2) tubulaire présente à sa deuxième extrémité un tronçon à filetage intérieur (24), et
le premier tronçon (61) de l'élément de maintien (6) présente un filetage extérieur pour être vissé dans le tronçon à filetage intérieur (24).

3. Vis à os selon la revendication 1, **caractérisée en ce que** la connexion entre l'élément de maintien (6) et le tronçon fileté (2) est réalisée sous la forme d'un ajustage serré.

4. Vis à os selon l'une des revendications 1 à 3, **caractérisée en ce que** l'élément de maintien (6) présente à son extrémité libre un élément (64) pour la prise extérieure ou intérieure d'un outil à visser.

5. Vis à os selon l'une des revendications 1 à 4, **caractérisée en ce que** la butée est prévue sous la forme d'un épaulement (62) entre le premier tronçon (61) et le deuxième tronçon (63) de l'élément de maintien.

6. Vis à os selon l'une des revendications 1 à 4, **caractérisée en ce que** la butée est prévue dans le tronçon fileté (2) tubulaire de la vis à os (1).

7. Vis à os selon l'une des revendications 1 à 6, **caractérisée en ce que** le tronçon (63) en forme de tige de l'élément de maintien (6) présente un filetage extérieur pour le vissage d'un écrou (83) depuis l'extrémité libre de l'élément de maintien (6).

8. Vis à os selon la revendication 2 et l'une des revendications 1 à 7, **caractérisée en ce que**
le tronçon fileté (2) tubulaire présente également à sa première extrémité un tronçon à filetage intérieur (23), ou le tronçon à filetage intérieur s'étend sur toute la longueur du tronçon fileté (2) tubulaire, et
la pointe (3, 5, 7) est vissable dans ledit tronçon à filetage intérieur (23) à la première extrémité du tronçon fileté (2).

9. Vis à os selon l'une des revendications 2 à 8, **caractérisée en ce que** le tronçon à filetage intérieur (23, 24) est réalisé avec un filetage métrique ou un filetage au pouce.

10. Vis à os selon l'une des revendications 1 à 9, **caractérisée en ce que** la pointe (7) est réalisée comme pointe autoperforante.

11. Vis à os selon l'une des revendications 1 à 10, **caractérisée en ce que** la pointe (5) est canulée.

12. Dispositif de fixation d'os (80), avec
au moins une vis à os selon la revendication 7 et l'une des revendications 1 à 11,
une plaque (81) pour la fixation d'un os avec un évidement (82) par où l'extrémité libre de l'élément de maintien (4, 6) est insérable, et
un écrou (83) à visser sur l'élément de maintien (4, 6) par l'extrémité libre de celui-ci et pour la fixation de la plaque (81) .

13. Dispositif de fixation d'os (90), avec
au moins une vis à os selon l'une des revendications 1 à 11, et
un clou centro-médullaire (91) pour la fixation d'un os avec un évidement (92) par où l'extrémité libre de l'élément de maintien (4, 6) est insérable.
